# EUROPEAN PATENT SPECIFICATION

(11) **EP 1 711 125 B1**
(45) Date of publication and mention of the grant of the patent: **16.11.2016**
(21) Application number: 05700838.5
(22) Date of filing: 12.01.2005
(51) Int. Cl.: A61F 2/06

(54) **MRI COMPATIBLE IMPLANT COMPRISING ELECTRICALLY CONDUCTIVE CLOSED LOOPS**
MRI-KOMPATIBLES IMPLANTAT MIT ELEKTRISCH LEITENDEN REGELSCHLEIFEN
IMPLANT COMPATIBLE IRM COMPRENANT DES BOUCLES FERMEES ELECTROCONDUCTRICES

(30) Priority: 12.01.2004 GB 0400571
(43) Date of publication of application: 18.10.2006
(73) Proprietor: Angiomed GmbH & Co. Medizintechnik KG, 76227 Karlsruhe (DE)
(72) Inventor: LAITENBERGER, Peter, Georg, Cambridge CB4 1AG (GB); SCOTT, Valerie Ann, c/o Scientific Generics Ltd., Cambridge, CB2 5GG (GB); WEBBER, Dominic George, Scientific Generics Ltd., Cambridge, CB2 5GG (GB); POOLEY, David Martin, c/o Scientific Generics Ltd., Cambridge, CB2 5GG (GB); JAMES, David Alun, Harston, CB2 5NX (GB); BLANK, Thiemo, Arnim, 68723 Plankstadt (DE)
(74) Representative: Hoffmann Eitle
(86) International application number: PCT/EP2005/000211
(87) International publication number: WO 2005/067816

(56) References cited:
- WO-A-99/10035
- WO-A-03/015662
- WO-A-03/075797
- WO-A-2004/071353
- US-A1- 2002 188 345

## Description

### Field of the invention

This invention relates to an implant having a plurality of electrically-conductive closed loops constituted from a plurality of portions that may be struts, the loops together forming apertured walls of a cage with an interior volume, the portions in any one said loop providing electrically-conductive pathways within which eddy currents are liable to be induced when the implant is subjected to a time-dependent external magnetic field, with each said loop having at least first and second said current pathways.

The archetypal implant considered here is a metal stent to be delivered in a radially compact configuration, transluminally, by a catheter, and then expanded into a radially larger deployed configuration, at a stenting site within a bodily lumen. However, the range of implants is steadily growing and the present inventors contemplate use of the invention in a wide range of implants other than stents. One particular example is a filter, such as a *vena cava* filter, and a range of grafts for bodily lumens. Thus, a Palmaz stent and a Gianturco "Z" stent have struts, but a Wallsten wire stent lacks such struts.

While stents exhibit a stent matrix built up from portions that resemble struts that terminate at ends corresponding to an abrupt change of direction, there are also stents made from a single smooth wire spiral, or a plurality of smooth wire spirals knitted or woven or braided together (like the child's plaything called the "Chinese finger"). Any oxide layer on the surface of the wire will inhibit or deny electrical conductivity from one wire portion to any other portion touching it.

Although polymers are of increasing interest and usefulness, metals continue to be important for stents, filters and stent grafts. In fact, the available range of biocompatible metals is relatively small. Stainless steel and nickel-titanium shape memory alloys are favoured for manufacture of stents and filters, but certain titanium alloys are widely used in particular implant applications. Tantalum is bio-compatible, radiopaque, and has an electrochemical potential similar to that of a nickel-titanium shape memory alloy, rendering it useful for radiopaque marking of nickel-titanium stents. Other bio-compatible materials, such as silver and gold, are also useful as radiopaque markers. Notably, all of these materials have good electrical conductivity and so a stent made of one of these materials is capable of functioning as a Faraday cage.

In this application, the term "Faraday cage" is intended to have its usual meaning, connoting a shield or screen surrounding a volume. The shield provided by a Faraday cage resists the penetration an electro-magnetic field into the interior volume and also prevents fields generated within the volume from exiting the volume. Thus, an implant which functions a Faraday cage can prevent an external time-dependent magnetic field from penetrating the interior volume of the implant.

### Background prior art

Magnetic resonance imaging (MRI) techniques are of increasing importance for imaging soft tissue structures in human and animal bodies. In these imaging procedures, the tissue to be examined is subject to a strong external time-independent magnetic field (the B₀-field) and superimposed on this time-invariant field is a time-dependent magnetic field (the B₁-field, typically a radiofrequency (RF) alternating field), which interacts with the nuclei of the soft tissue structure most often the protons (H⁺) in the nuclei. If one superimposes on these two B-fields a further magnetic field that has a field strength gradient across the field of view, then one can extract positional data from the field of view, and build an image.

Clearly, it would be useful to have an MRI image of the biological structures or fluids inside the lumen of a metal stent. However, when one takes an image of a field of view that includes a conventional metal stent, little information can be derived from the portion of the image corresponding to the stent lumen. The metal stent functions as a Faraday cage, to shield the lumen from the B₁-field, with the result that the lumen of the stent is not rendered clearly visible in an MRI image.

It is an object of the present invention to provide implants that define an interior volume and which make that volume and zones adjacent to the implant available for MRI imaging, while retaining a useful mechanical performance of the implant.

In MRI apparatus, the B₁-field can induce eddy currents in any closed loop of electrically-conductive material whose plane does not lie parallel to the direction of its imposed time-dependent magnetic field.

A metal stent to be delivered transluminally can be made from wire stock, tube stock or sheet stock, and often is a line of metal stenting rings arranged along the long axis of the stent. Irrespective of whether the stent is (to name but a few examples) a knitted stent made of wire, or a laser-cut stent made of tubular material or sheet material formed into a tube, the rings may be formed of metal struts which zig-zag around the circumference of each of the rings. Adjacent rings may be connected at one or several locations, herein called bridging portions. Within the overall stent structure defining the walls of the stent lumen, what one may call the stent matrix, one typically finds a multiplicity of closed electrically-conductive loops which need not be confined to within one stenting ring, but can include portions within two or more of the stenting rings. Eddy currents are liable to be induced in these closed loops of the stent matrix, whenever the metal stent is within the field of view of an MRI machine and the time-dependent magnetic field vector passes through the area defined by the closed loop. The eddy currents induced in the closed loop generate a magnetic field, which is what delivers the Faraday cage effect, defeating the ability of the MRI apparatus to yield a useful image of the matter within the stent lumen.

Accordingly, there have been various proposals to reduce or eliminate the induction of eddy currents in stents to be subject to the fields within an MRI machine.

An expandable metallic stent said to be MRI-compatible is disclosed in published US Application No. 2002/0188345 A1. The stent has discontinuities of non-conducting material. These eliminate electrically conducting paths in the stent rings and loops. The discontinuities are said to facilitate MRI-imaging of tissue within the stent lumen. The non-conducting material can be selected from various materials, such as adhesives, polymers, ceramics, composites, nitrides, oxides, silicides and carbides. The discontinuity is preferably shaped such that, during expansion, the discontinuity is located where a compressive stress is suffered by the discontinuity. The discontinuities are advantageously placed circumferentially along the stenting rings.

US-A-6,280,385 discloses a stent which has at least one passive resonance circuit with an inductance and a capacitance whereby its resonance frequency is essentially equal to the frequency of the applied RF-field of the MRI imaging machine. The stent is essentially acting as a secondary resonance coil in the MRI system. A change in the signal response is thus produced in a locally defined area in or around the stent which is imaged in spatial resolution.

A metallic endoprosthesis is disclosed in WO 03/015662 which is said to cause no significant artefacts on images taken by magnetic resonance tomography (MRT), as a result of a combination of the production materials with a special design. The endoprosthesis is made from a material with a magnetisability similar to human tissue. The design of the endoprosthesis is such that the members or wires of the endoprosthesis run extensively along the longitudinal axis of the endoprosthesis, without forming a closed circuit in a plane which is essentially perpendicular to the long axis of the endoprosthesis. As endoprosthesis material, copper, gold, copper-gold alloys and silver-palladium alloys are said to be suitable. The design of the endoprosthesis includes a spine to which annular elements performing the function of the endoprosthesis are attached via electrically non-conductive links. The annular elements do not exhibit closed electroconductive loops in a plane perpendicular to the spine.

In Applicant's WO 03/075797, a medical implant in the form of a stent is described in which adjacent stenting rings are connected at bridging portions. The bridging portions comprise conductivity breaks to reduce, or even eliminate, the induction of eddy currents when the implant is exposed to the B₁-field of a MRI machine.

However, there are disadvantages associated with each of these prior proposals. The present inventors have started from first principles, and looked for ways to make the lumen of a metal stent available for imaging in an MRI machine. One way to reduce the amount of eddy currents flowing is to increase the electrical resistance of the stent itself. One could make the stent from less conductive material, make the current flow paths longer, or reduce their cross-sectional area, as by replacing short thick struts of a stent matrix by longer thinner and more convoluted connectors. However, this has consequences for the mechanical strength of the stent, and for the steps involved in its manufacture.

In another approach, one might inhibit the flow of eddy currents through a stent matrix by eliminating, or reducing, the number of longitudinal electrical connections between adjacent stenting rings that extend around the stent lumen. This is a realistic approach within the field of stent grafts, where structural integrity along the length of the implant can be provided by the covering of the stenting rings. For an example of a stent graft with spaced stenting rings see WO 96/28115. In bare stents, however, separating the stenting rings has consequences for the mechanical integrity of the stent, whether it will survive the rigours of assembly and delivery, and how feasible it is to manufacture such a stent.

Thus, the present inventors looked into other possibilities to render more visible to MRI imaging the local volume of an implant and zones adjacent to the implant.

### Summary of the present invention

According to the present invention there is provided an implant, as defined above, and characterised by first and second current pathways that are arranged in any one closed loop such that, in any particular electro-magnetic field, the direction of the eddy current that would be induced in the second current pathway is the reverse of the direction of the eddy current that would be induced in the first current pathway, so as to mitigate the tendency of the implant to function in said magnetic field as a Faraday cage.

The insight which the inventors have brought to the problem is that an arrangement of conductive current pathways in a loop which is liable to give rise to eddy currents is not a disadvantage, provided that there is, at the same time, another part of the same electrically-conductive closed loop that is liable to induce eddy currents of equal strength, but in the opposing direction. In this way, the aggregate flow of current in the closed loop is zero (or near zero), and so the Faraday screening effect preventing the interior lumen of the implant from being imaged by MRI is at least mitigated, or even eliminated.

To take a very simple example, imagine a simple circular loop of conductive material, arranged in a plane transverse to an external time-dependent magnetic field. Eddy currents will be induced in the loop. However, imagine holding the loop at two diametrically opposed points, and then rotating one point through 180° relative to the other point. Out of the single loop in a given plane, one has created a figure of eight in the same plane, with portions of the single closed loop crossing over each other where the two lobes of the figure of eight have their crossing point. With the two crossing portions electrically isolated, so there is no short circuit at the cross-over point, imagine what will be the effect within the conductive material of the figure of eight loop, when exposed to the same external magnetic field. In both lobes, there will be a force to create eddy currents to flow in the "same" direction (clockwise, or anti-clockwise, depending on the direction of the magnetic field). However, were eddy currents to flow in the same direction in both lobes, they would meet "head-to-head" at the crossing point between the two lobes. Neither can flow, because they are equal and opposite, when the two lobes have equal area. Accordingly, no current flows. This principle holds true, no matter what the angle is between the plane of the loop and the direction of the incident time-varying electro-magnetic field.

To grasp the underlying idea of the invention it may be helpful to imagine the "figure of 8" shaped piece of paper with its top surface green and its undersurface red, and the direction of the B₁ field normal to the plane of the paper. The B₁ field "sees" two green circles, each being one of the lobes of the "8". But when one lobe is rotated 180° with respect to the other, the B₁ field sees one red and one green circle, the two circles being co-planar and having the same area. The eddy currents that would be induced to flow around the periphery of the red circle cancel those that would flow around the periphery of the green circle.

It is this simple principle that has informed the development of embodiments of the present invention. The inventors have given this concept the name "balanced coil".

Although the balanced coil concept is at the heart of the present contribution to the technical field, nevertheless a second effect, which we call "inductive inhibition", has been found to be important in the facilitating of imaging of stent lumens.

Let us revert to the simplest case of two parallel wires of electrically conductive material. If an AC current flows in one of these wires then, by a process of induction, eddy currents will be caused to flow in the second wire but in the opposite direction to that in the first wire. Extending this principle, when a number of wires run close to and parallel to each other, then eddy currents are caused to flow in neighbouring wires due to the current in any particular wire. The net effect of all these additional eddy currents is that the effective resistance of each wire is increased. This effect is often noted in transformer and inductor winding and it is commonly termed the "proximity effect". This effect can be used to deliberately increase the resistance of all the loops in the implant, by making all the loops as convoluted as possible and by running the loops as close to each other as possible, so that the proximity effect is maximised. This has the effect of decreasing the currents that can flow in that implant, so that the visibility of the implant lumen is improved in the MRI image.

Turning now to realistic stent matrix structures, one can pursue the figure of eight idea into the formation of an implant from a plurality of closed electrically conductive loops, with each of these closed loops being made up of a string of lobes arranged in line and extending in that line along the length of the stent cylinder parallel to the axis of the stent cylinder. The lobes of this line are interspersed by cross-over points where the conductor of the loop crosses over itself when advancing from one lobe through the crossing point to the adjacent lobe. One envisages that there will be electrical insulation between the two electrical conductors that cross over at each cross-over point. Evidently, the multi-lobe closed loop can be created by successive 180° rotations of one end of the loop relative to the other, and these successive rotations can be all in the same direction, or alternately in one direction and then the other direction to create successive cross-over points, or in some other format. Either way, using our "coloured paper" visualisation, the successive cross-overs produce alternate red and green lobes down the length of the stent.

In an embodiment where all lobes have equal area, there should be an even number of such nodes. However, one can envisage embodiments in which a smaller number of large area nodes are arranged with a larger number of cancelling small area nodes, so as to deliver an eddy current cancellation effect, in aggregate. Reverting to our image of paper with one side green and the other red, the B₁ field should "see" the same aggregate total area of red paper as green paper.

These different formats will influence the mechanical properties of the resulting implant. However, a feature of note in all of these constructions is that there is a double thickness of the electrical conductor, at each cross-over point. In many medical implants, there is a premium on keeping the wall thickness of the implant, between bodily tissue and the interior volume of the implant, as small as possible, and as uniform as possible, so that double thickness cross-over points may be regarded negatively.

With the present invention, various stent matrix constructions known, as such, within the state of the art, can be modified to take some advantage of the benefits of the inventive concept.

For example, the present Applicant makes self-expanding stents from tubes of nickel-titanium shape memory alloy, using a laser cutting tool to cut slots in the wall thickness of the metal tube, in a configuration that allows the tube radially to expand when angles open up between struts that are created in the wall thickness of the metal tube by cutting a multiplicity of slots through the wall thickness. Typically, the slots are all parallel to the long axis of the metal tube, so that when the radius of the slitted tube is expanded, there is evident a sequence of zig-zag struts extending around the circumference of the expanded metal tube, in a sequence of zig-zag stenting rings stacked along the long axis of the metal tube.

The flexibility of the stent thus formed varies with the number of links or bridges that connect each of the stenting rings to the axially adjacent stenting ring next to it along the length of the stent. Thus, the stent exhibits closed electrically conductive loops around the lumen of the stent, and other closed loops that extend along the lumen within the annular envelope of the stent. One can appreciate that, when adjacent stenting rings are connected by only one link at only one point of the circumference of the metal tube, there is considerable freedom for relative bending movement of one stenting ring relative to the next adjacent stenting ring. Indeed, in the ultimate, one can make entirely separate stenting rings and sandwich them between two layers of tubular graft material so that the flexibility of the resulting stent graft, between two adjacent stenting rings, is limited only by the stiffness of the graft material, and not by the material of the stenting rings. However, for an uncovered stent, it is typical to provide a number of links between adjacent stenting rings that is less than eight per circumference, and often four per circumference.

Now, such a stent exhibits a multiplicity of closed loops of electrically-conductive material lying within the externally imposed time-dependent magnetic field of an MRI imaging machine. There are closed conductive loops within each stenting ring, and other closed conductive loops that include portions of different adjacent stenting rings. Eddy currents will tend to flow in such loops. The precise distribution of eddy currents varies with the distribution of links or bridges within stenting rings and between adjacent stenting rings. However, previous proposals to expose the stent lumen to MRI imaging have involved installation of one or more conductivity breaks somewhere on the circumference of each one of the closed loop stenting rings. The proposal of the present inventors is different.

Suppose that each closed loop could be modified in some way (as the above single loop was modified into the figure of eight shape), namely, to create within any one closed loop portions in which the eddy currents flow in one direction which exactly cancel the effect of other portions in which the eddy currents would tend to flow in the opposite direction. The aggregate flow of eddy currents within such a closed loop ought to be zero.

It will be recalled that, in the figure of eight, by providing that the two lobes, i.e. both halves, of the figure of eight shaped loop seen by the time-dependent B₁-field have equal area, lie in the same plane, and are cut by the same direction and density of incident field, each lobe "cancels" the other, so that the total flow of eddy currents induced in the figure of eight shaped loop is zero.

In the discussion above, the figure of eight is flat and the B₁ field is perpendicular to the plane containing the two lobes. In a real stent, however, the lobes are not flat but are found within the annulus of the stent matrix. Looking end-on at the annulus and taking the top of the circle as a reference point "N", the lobes of the figure of eight described above lie on a line parallel to the stent axis that runs the length of the axis and through reference point N.

Reverting to the piece of paper with red and green faces, now imagine the figure of eight draped over the stent cylinder, like a saddle on a horse with the waist of the figure of eight co-inciding with reference point N, and one lobe on each side of the cylinder, the two lobes almost touching at the point "S" on the other end of the diameter from "N". Think of a clock face, in which N is 12 o'clock. Then S is 6 o'clock.

Now consider incident B₁ fields. One that is parallel to the stent axis "sees" no green or red at all. A B₁ field passing through the cylinder perpendicular to the vertical diametral plane that includes reference points N and S "sees" first an area of one colour, as it enters the stent cylinder but then, as it exits the cylinder, it "sees" in the other colour an area of equal size, on the other side of the vertical diameter from the first area.

Thus, the figure of eight "draped" or "wrapped" around the stent annulus also is a potential "balanced" coil. Note that there should be no cross-over of the conductive pathways at the waist of the figure of eight, when advancing from one lobe to the other (if there was, then the B₁ field perpendicular to the stent axis would "see" the same paper colour on both of the two lobes through which it successively passes, and then the two lobes would re-inforce each other in the creation of eddy currents).

An embodiment that has no requirement for the double thickness of a cross-over point is distinctly advantageous in stent design.

We have looked at two of the three orthogonal directions of an incident B₁ field. The third is the case of a B₁ field in the vertical diameter that includes reference points N and S and transverse to the long axis of the stent cylinder. It passes through an area corresponding to the waist of the figure of eight, at N, but no such area at S, where the two lobes approach each other but are not electrically joined. If the waist area is large, one may then expect some eddy currents in consequence, because that area is not "balanced" in the sense explained here.

In reality, with stent strut matrices resembling those of conventional stents, the "waist" area can readily be confined to an area that is vanishingly small. Likewise, at the point "opposite" the waist, where the two "wrapped" lobes face each other, they can readily be separated by the negligible distance of a film of electrically insulating material.

Evidently, such a "wrapped" balanced coil can have i) more than two lobes, and ii) offers just as much stenting performances a stent with an analogous strut matrix but which does not comprise any "balanced" coils as described herein.

The "wrapping" of a balanced coil around a stent cylinder is not confined to wrapping a coil in a plane transverse to the stent axis. Instead one can arrange successive lobes of an endless loop on a spiral path around the stent axis. For maximum effectiveness, as explained above, the loop should consists of a number of lobes where the area of the lobes cut by the B₁ field on one side of the stent cylinder is balanced by an equal and opposite aggregate lobe area on the other side of the stent cylinder. Two lobes spaced apart around the circumference of the stent annulus by 180° is but the simplest example of the concept.

With stents created from a laser-cut tube, the balanced coils will exhibit a plurality of lobes and can be arranged transverse to the stent axis, or to spiral along it. In particular, one preferred arrangement is referred to in this application as the "winding" concept. According to the winding concept, each such endless loop spirals around the interior volume, preferably (but not necessarily) by an integral number of turns around the long axis of the stent.

Thus, in a typical stent, a plurality of these "winding loops" define a cage which has a central longitudinal axis and defines an interior volume which is tubular and centred on that axis. The tubular form will often be cylindrical, but not necessarily so. It might, for example, have flared ends. In the circumference of the cage, at any one cross-section transverse to the length of the stent, there are to be found co-operating portions of at least two of the winding loops that make up the cage.

A major advantage of both the "figure of eight" and the "wrapped" or "winding" loops is that the cancelling effect is independent of the orientation of the stent with respect to the direction of the B₁-field. Thus, a stent which embodies the concept does not form a Faraday cage whatever its orientation with respect to the B₁-field. Hence, the detrimental screening effects due to the Faraday cage are at least mitigated, if not eliminated. This renders such a configured stent particularly attractive, because the lumen or the interior volume of the stent can be imaged using an MRI imaging machine irrespective of the orientation of the stent with respect to the direction of the B₁-field.

For structural integrity, there can be connections between portions within each winding loop, and other connections between adjacent winding loops, and all of these connections should include conductivity breaks. There will be a premium on reducing the number of electrically-conductive links. The ability to install conductivity breaks in these links will give the designer more freedom to manipulate the mechanical properties of the implant thus formed.

If these links are not to include conductivity breaks, then they can simply be formed of the material of the metal tube from which the implant is created. Otherwise, one can envisage mechanical links such as by a hook and eye formation. If a link between adjacent winding loops is to include a conductivity break, then it might be achieved by a layer of adhesive composition between cooperating form-fitting portions of adjacent winding loops or, in a mechanical link that allows relative movement of the two portions forming the link, an electrically insulating coating on one or both of the cooperating link portions. One way of providing such an insulating coating is deliberately to build a sufficiently thick and robust oxide layer. Another way is by local deposition of a material such as diamond-like carbon on that part of the surface of one link portion that contacts a link portion of the adjacent winding ring.

For a better understanding of the invention, and to show more clearly how the same may be carried into effect, reference will now be made, by way of example, to the accompanying drawings.

### Brief description of the drawings

Fig. 1 is a diagram to show the principle of balancing eddy-currents induced in two portions of a closed loop;
Fig. 2 is a schematic view of part of an implant realizing the principle shown in Fig. 1, opened out from tubular to a flat configuration;
Fig. 2A is a schematic view of the implant of Fig. 2, not opened out flat;
Fig. 3 is a schematic view of a balanced implant embodying the invention, that has been unwrapped along the long axis of the implant, from tubular to a flat configuration;
Fig. 3A is a schematic view of the implant of Fig. 3, not opened out flat;
Fig. 4 is a schematic view as in Fig. 3, and showing four balanced strut loops with intra- and inter-loop insulating connections;
Fig. 4A is a schematic view of the implant of Fig. 4, not opened out flat;
Fig. 5 is a schematic view as in Fig. 3, of a geometry that can be used to produce closed balanced loops with intra- and inter-loop insulating connections;
Fig. 5A is a schematic view of the implant of Fig. 5, not opened out flat;
Fig. 6 is a perspective view of two stenting rings composed of struts arranged in zig-zag form, and in their radially small configuration prior to deployment, and ring-to-ring connecting portions;
Fig. 7 is a perspective view of the two stenting rings shown in Fig. 6, but separated from each other;
Fig. 8 to 10 are schematic views of constructions for connecting portions;
Figs. 11 to 24 are isometric views of various joint constructions, in which:
   Figs. 12 to 14 include a retaining strut;
   Figs. 15 to 18 include a cord link;
   Figs. 19 to 22c exhibit a shaped joining piece; and
   Figs. 23 and 24 exhibit a mechanical interlock between co-operating portions of the adjacent parts of the implant;
   Fig. 25 is a copy of Fig. 4 from Applicant's WO 01/32102; and
   Fig. 26 is a portion of a stent matrix, in side view, showing two forms of insulating joint in an embodiment of the present invention.

### Detailed description of the preferred embodiments

Typically, a stent is made from metal wire, or a tubular matrix of metallic struts can be formed from seamless tubular material, or from flat sheet material rolled up. Although the following description only refers to transluminally-delivered, expansible stents, the principle of the present invention may be applied to medical implants in general other than transluminal stents, such as implants installed in a bodily lumen by open surgery, filters such as vena cava filters, fluid-flow measuring devices, valves such as heart valves or venous valves, etc.

To prevent eddy currents from flowing, one alternative has been to eliminate conducting links between adjacent stenting rings that form the stent. This, however, physically weakens the stent so that it may fail to operate properly, e.g. it may not deploy correctly and once deployed it may not be strong enough to withstand the arterial pressures. Connecting the stenting rings by means of insulating joints brings extra manufacturing tasks and extra burdens of compliance with regulatory authorities.

The present invention aims to reduce eddy currents, without unacceptable loss of performance, by designs that are electromagnetically "balanced" with respect to an external time-dependent MRI-field. In the following, this category of stent is referred to as a "balanced coil" stent. The aim is that any electrically-conductive closed loop within the stent has opposing portions, such that eddy currents that would be generated in one portion are opposed by eddy currents that would be generated in another portion of the closed loop. Thus, the two portions of the loop cancel, or balance each other, and no eddy currents flow.

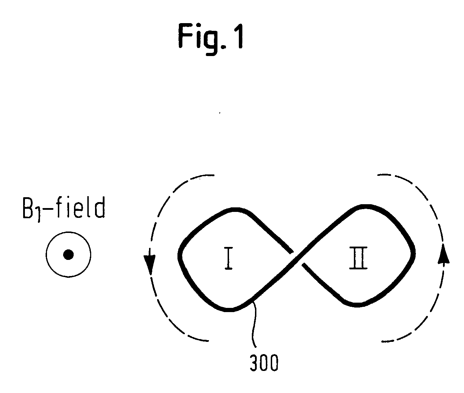

Fig. 1 illustrates one example of such a geometry ("figure of eight") for reducing, even eliminating, eddy-currents being induced in a metallic stent matrix when being exposed to a time-dependent magnetic field.

The time-dependent magnetic field (B₁-field) of an MRI imaging apparatus in Fig. 1 is perpendicular to the plane of view and going into the page. A conducting loop 300 (solid line) which is part of a metallic stent matrix can be divided in two portions, which we may call "lobes", namely labelled portion "I" and labelled portion "II". Induced currents in loop 300 are indicated by dotted lines with arrows.

The B₁-field induces anticlockwise currents in both portions of loop 300 but, due to the geometry of loop 300, an anticlockwise current in portion "I" would flow as a clockwise current in portion "II", and vice versa. Thus, the two currents oppose each other and the total amount of current flowing through loop 300 is reduced, even eliminated. Use of such a "balanced" coil in the matrix of a stent can reduce, even eliminate, the detrimental Faraday cage screening effects suffered by conventional stent matrix designs in a MRI apparatus.

With the present invention, a stent can be made up from a number of separate loops that are insulated from each other, and with each of these loops exhibiting a "balanced" configuration, so that the total electro-magnetic effect of the stent cage is reduced, even eliminated.

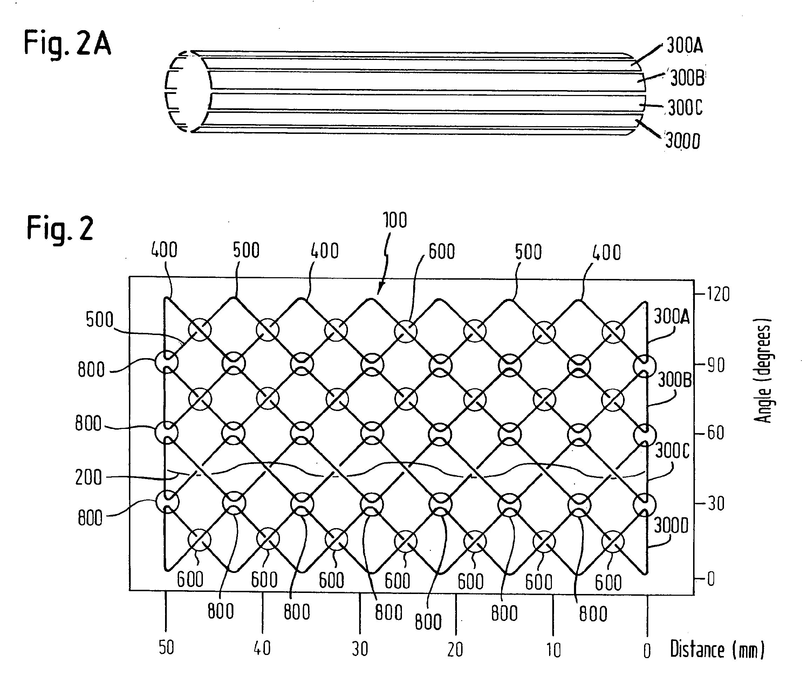

Fig. 2 illustrates a schematic of part of a stent design in which each of twelve multi-lobe closed conducting loops is elongate parallel to the length of the stent lumen. Thus, each of the loops forming the stent matrix are "balanced". The horizontal axis in Fig. 2 represents the distance from one axial end to the other axial end of the stent, whereas the vertical axis represents the angle around the circumference of the stent. Thus, Fig. 2 depicts the stent in the flat configuration. Because the angular range goes from 0° to 120°, only a third of the full circumference of the tubular stent is shown opened out flat in Fig. 2.

Each of four individual loops 300A, 300B, 300C, 300D has a length direction parallel to the longitudinal axis of the stent and extends the full length of the stent 100. Fig. 2 shows only part of the stent matrix. Altogether, the stent has twelve of these individual loops, arranged regularly around the circumference of the stent so the stent in transverse section exhibits a regular dodecahedron configuration. Fig. 2A shows this, and reveals the 120° circumferential extent of the stent matrix portion shown in Fig. 2.

The upper two loops 300A, 300B in Fig. 2 illustrate one embodiment of the twisting required to achieve the "balancing" effect, and the lower two loops 300C, 300D show an alternative embodiment of the twisting. In the two loops 300C, 300D, advancing along the wire of one side of the loop from one end of the stent to the other, one side is always "over", or always "under", the wire of the other side at each cross-over point. However, in loops 300A, B, making the same advance, one threads alternately over and under the wire of the other side at each successive cross-over point. In working embodiments, however, a stent will likely feature all loops with the same twisting pattern, either that of loops 300A, B or that of loops 300C, D.

In loops 300A and 300B, length portion 400 lies at cross-over points 600 alternately above and below length portion 500. Loops 300A, 300B thus form what one might term a "braided" or an intra-engaged stent loop, which can be formed by unidirectionally twisting one end of a wire rectangle relative to the other.

Conversely, the cross-over arrangement of loops 300C, 300D might be achieved simply by "folding" half the length of each closed loop over the other half, so that all the zig-zags of the second half of the loop length overlie the zig-zags of the first half of the length.

The circles shown in Fig. 2 indicate insulating connections 600 at which portions 400 and 500 of each individual loop are fixed to each other in order to prevent unwanted relative movement of such loop portions. Given the "braided" arrangement of loops 300A, B, there might be a lesser tendency to unwanted relative movement, so a lesser need to fix each cross-over point with a connection.

The loops are held together by loop-to-loop electrically-insulating connections 800, whereby it may be possible to omit the loop connections 600 within each loop. Portions 400 and 500 of a single closed loop should nevertheless be insulated electrically from each other at each cross-over point along the length of that loop.

Another possibility for not having to use the intra-loop connections 600 is to utilise a strand 200 that is woven above and beneath loop sections 400 and 500 at the electrically-insulating cross-over points 600, and attach both ends of the strand 200 to the axial ends of each of the loops to hold each loop together.

Various methods of insulating the cross-over points are feasible, such as inserting an electrically non-conductive element between the overlapping portions of each of the loops, or coating the overlapping portions with a layer of non-conductive material. It is also contemplated to reduce the thickness of each of the overlapping portions locally at the cross-over points, in order to avoid a double wall thickness of each of the loops at such cross-over points.

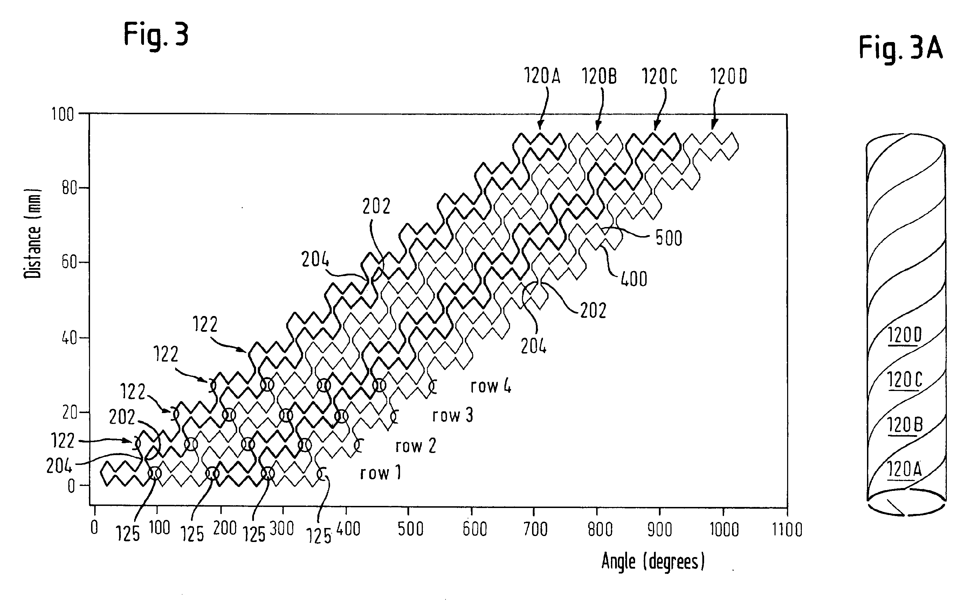

Another embodiment of a "balanced" design is here referred to as a "winding" stent, and is schematically illustrated opened out in Fig. 3, and not opened out flat in Fig. 3A.

The "winding" stent design consists of four closed conducting strut loops 120A, 120B, 120C and 120D each of which winds spirally around the long axis of the stent. The vertical axis in Fig. 3 indicates the length direction of the stent. The horizontal axis in Fig. 3 indicates the winding angle by which each of the loops 120A, 120B, 120C and 120D is wound around the long axis of the stent. This "winding" stent approach is, of course, not limited to four strut loops, and the winding angle is not limited to that shown in Fig. 3.

In Fig. 3, four loops 120A, 120B, 120C and 120D are shown which are insulated from one another at connecting joint positions 125 indicated by circles. Each loop is helically wound around the longitudinal axis of the stent, i.e. they spiral around the stent. In this instance, the length of each loop is such that its spiral around the stent by 720° as shown more clearly in Fig. 3A. To minimise generation of eddy currents irrespective of the direction of the incident magnetic field, the winding angle should be an integral multiple of 360°. The illustrated stent is built up from struts 4 mm long, with a length of 96 mm (24 struts). A zig-zag stenting ring of 48 struts also extends around the 360° circumference of the stent lumen, anywhere along the length of the stent. For other stent lengths, other strut numbers, and other loop lengths and numbers, will likely be indicated.

Each loop consists of a multiplicity of metal struts joined end-to-end to create a zig-zag pattern which winds around the longitudinal axis of the stent. Those portions of each of the four loops 120A, 120B, 120C and 120D which are located at the same distance from, for example, the lower axial end of the stent matrix are indicated in Fig. 3 by row 1, row 2, row 3, row 4, etc. The portion of loop 120A at row 1 and the portion of loop 120A at row 4 are at opposite sides of the tubular stent matrix, i.e. they are disposed 180° apart from each other. Two adjacent rings of 24 struts form a stenting ring of 48 struts, or "row", with a 360° circumference, and each such row includes struts from each of the four winding loops. The stent has 12 such rows, and 12 stenting rings within its overall length of approximately 96 mm, so each ring of struts has a length of 4 mm, each row a length of 8 mm (and each stenting ring a length of 8 mm).

In a conventional stent made from a tube, each stenting ring is a closed electrically-conductive loop. By installing electrically-insulating breaks between the winding loops of the present invention, one avoids the creation in the stent matrix of the electrically-conductive stenting rings of the conventional stent.

Regarding loop 120A, the portion at row 1 and the portion at row 2 are electrically connected with a pair of parallel links 202, 204 which are insulated from one another. There are analogous electrically-insulating links 125 between facing portions of adjacent closed loops 120A, B, C, D.

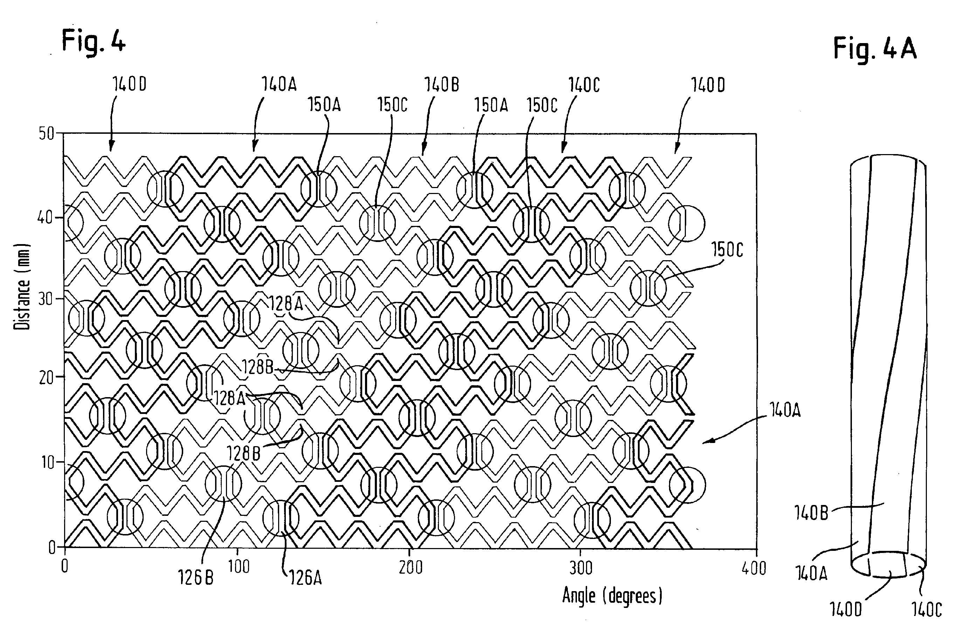

An alternative way of realising a "winding stent" is illustrated in Fig. 4 which shows a stent consisting of four winding loops each of which has six lobes. Fig. 4 shows only a portion of the length of the implant. The complete implant needs to be of a length such that each lobe of each loop has an equal and opposite "partner" lobe that is disposed 180 degrees apart from it on the opposite side of the stent.

Fig. 4 is a schematic showing four separate loops 140A, 140B, 140C and 140D which are helically wound in longitudinal direction around the stent axis. The vertical axis indicates the distance from one axial end of the stent to the other, whereas the horizontal axis indicates the angle by which each of the loops 140A, 140B, 140C and 140D is wound around the stent axis.

Each loop is made up from zig-zag metallic struts joined end-to-end with full electrical conductivity. The struts that compose a single zig-zag line of one of the loops are electrically connected to the next adjacent zig-zag line of the same loop by a pair of parallel struts insulated from each other and marked in Fig. 4 by circles 126B. In addition, portions of each winding loop are connected to portions of the adjacent winding loop in the same row by a pair of parallel struts, again indicated by circles 126A. In other words, the stent matrix shown in Fig. 4 has both intra- and inter-loop connections, all of which include an electrical conductivity break and are indicated by circles in Fig. 4.

The intra-loop insulating connections within each of the loops and the inter-loop connections between adjacent loops lie on a path which spirals around the long axis of the stent (in Fig. 3 they lie on equidistantly spaced diagonals). Following a hypothetical path from one circled insulating joint to a neighbouring circled insulating joint (see the dashed lines in Fig. 4), at least one pair of struts 128A, 128B which has been cut in transverse direction is encountered, quite in contrast to the design shown in Fig. 3. These additional cuts can increase the flexibility of the stent matrix both in longitudinal and transverse direction to the stent matrix.

Fig. 4A reveals more clearly that each winding loop of Fig. 4 wraps around less than one full turn of the circumference of the stent matrix cylinder, in fact, only a quarter of a turn, from one end of the cylinder to the other. An implant approximately four times the length of the portion of Fig. 4 satisfies the requirement for balancing of eddy currents - when each lobe of the loop has a corresponding equal and opposite lobe to balance the currents.

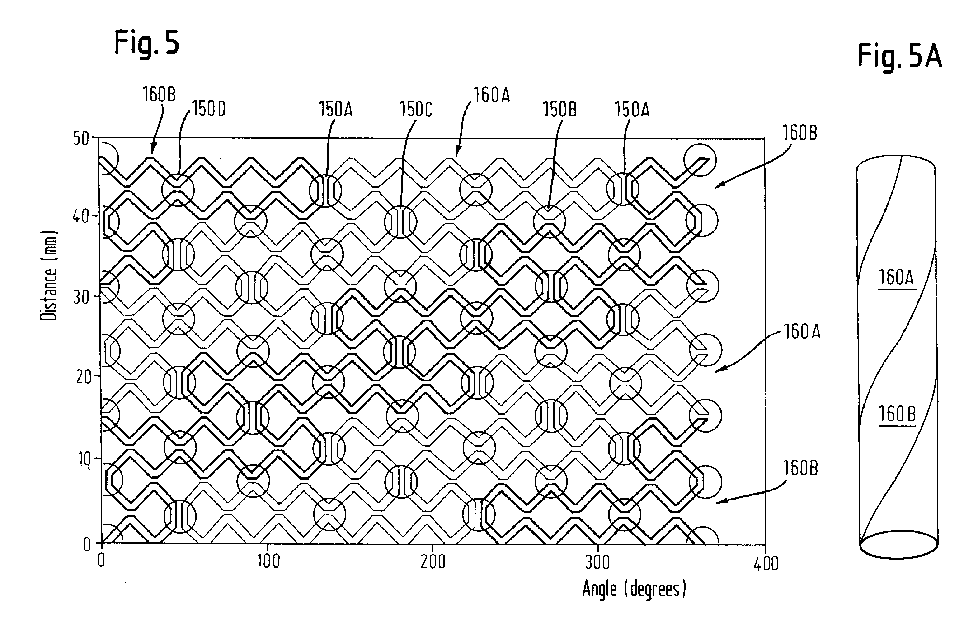

Referring now to Fig. 5, this drawing figure illustrates a "winding" stent design with only two closed loops 160A, 160B which spiral their way around the long axis of the stent. Again, the vertical axis in Fig. 5 indicates the distance along the axial length of the stent, whereas the horizontal axis indicates the angle through which each of the loops has progressed around the long axis of the stent.

Fig. 5A reveals that each of the two winding coils of Fig. 5 extends around 450° of circumference of the stent cylinder. The stent length is 48 mm, made up of 6 stenting rings. Evidently, a reduction of length of the stent cylinder, to 40 mm, and 5 stenting rings instead of 6, would leave each winding coil extending around exactly one integral turn around the stent lumen. Nevertheless, the winding coils of Fig. 5 deliver a "balanced" configuration because the lobes of each winding coil exhibit equal and "opposite" areas in terms of eddy current generating capacity.

The small circles superimposed on the strut matrix in Fig. 5 indicate both insulating intra- and inter-loop connections. In contrast to the stent design shown in Fig. 4, transverse insulating intra- and inter-loop connections are provided which impart additional stiffness to the stent design, in particular in longitudinal direction. By selectively incorporating longitudinal and transverse insulating intra- and inter-loop connections, the structural stiffness of the stent can be altered as required, yet the effects of a "balanced" design are maintained.

The features shown in Fig. 4 and Fig. 5 are not limited to a stent matrix comprising four loops and two loops respectively, but can be modified and combined, if required.

Below is a description of how insulating connections, in general, and in particular the insulating intra- and inter-loop connections can be made, and reference is hereby made to Applicant's earlier International Application Nos WO 01/32102, WO 02/15820, WO 02/047575 and WO 03/075797, all hereby incorporated by reference.

In the illustrated embodiments, the stent is made from Nitinol®, a nickel-titanium shape memory alloy. In other embodiments, the stent could be made of stainless steel, or any other biologically compatible conducting material capable of performing a stenting function.

It is conventional to form the lattice pattern of Nitinol® stents by laser-cutting. Cutting the frusto-conical mating surfaces of the body portion of the stent is achieved by aligning the laser in the normal, i.e. radial direction, thus intersecting the long axis of the stent tube. Once the slits in the workpiece of the stent tube are cut, most but not all of the vertices axially connecting two adjacent rings of the stent tube are severed, and only a few remain connected in order to maintain an integral tubular stent structure. The smaller the number of connected vertices, the greater the potential the stent has to bend out of a straight line as it is advanced along a tortuous path to the site of stenting. In addition, the flexibility of the stent after deployment is increased as well.

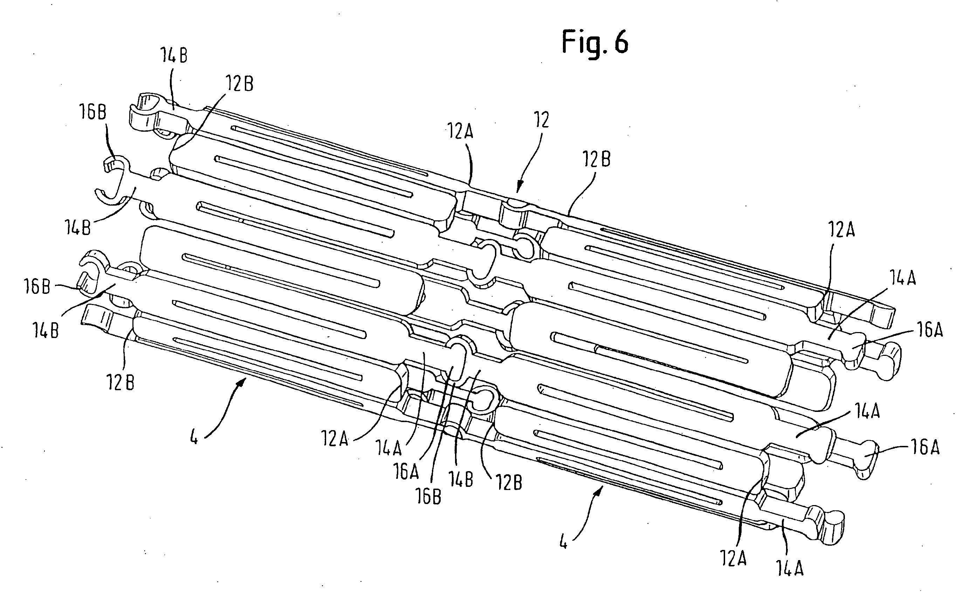

As can be seen in Fig. 6, taken from earlier WO 03/075797, the bridges 12 connecting two adjacent vertices 12A, 12B at two ends of a stent ring 4 facing each other, have a non-zero length, which, in turn, renders the overall structure in a radially compressed configuration more flexible, so that it can be more easily advanced along a tortuous path within a body lumen.

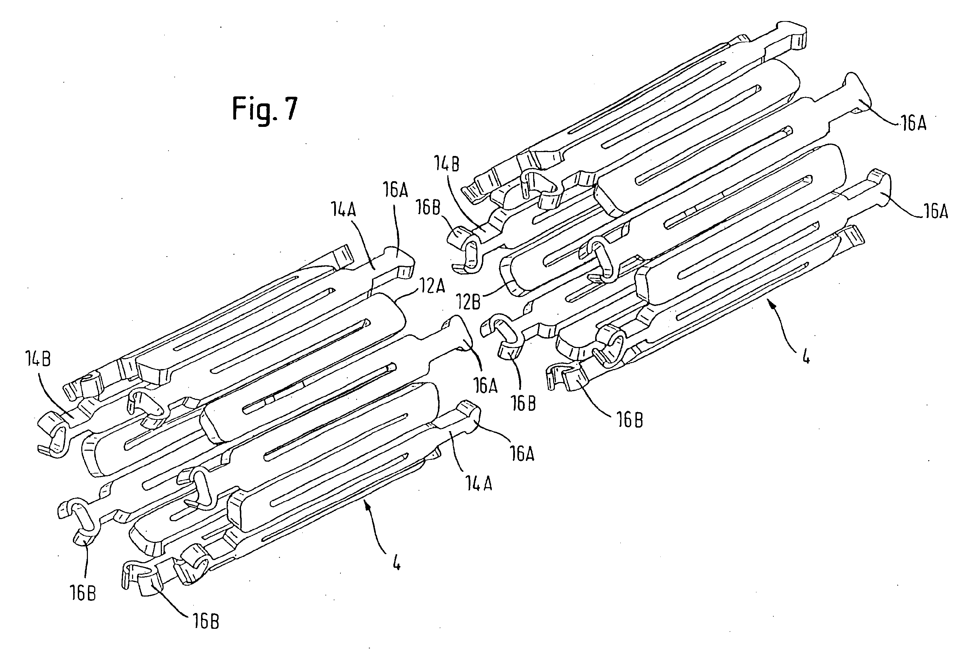

Fig. 7 shows in more detail how individual rings 4 of the stent are connected with one another. Here, the stent cylinder is shown in its radially compact disposition. In particular, attention is drawn to the constructional details of the connection points, i.e. the bridges connecting the vertices 12A, 12B of two adjacent rings 4. Figs. 6 and 7 illustrate two zig-zag rings 4, which comprise bridge struts 14A, 14B at both axial ends of each of the rings 4. All of the bridge struts include a straight portion provided for enhancing axial flexibility of the stent tube.

The protruding portions of the bridge struts 14A, 14B can be classified into male portions having an arcuate head portion 16A and female portions having an arcuate recess portion 16B. The female portions comprise rebated internal abutment surfaces to receive the complementary arcuate male head portion. Both male and female portions are frusto-conically shaped, a consequence of the laser-cutting process, as described in WO 03/075797. Thus, due to the complementary-shaped male and female portions, they represent a form-fit when connected together which gives the male and female portions excellent attachment security and the bridges are thus self-centering and self-aligning.

Furthermore, the luminal and abluminal major surfaces, out of which the arcuate head portion and the arcuate recess portion are formed, share the same radius of curvature as the major surfaces of the zig-zag rings. This, however, is not necessarily the case when the stent cylinder is initially laser-cut from flat sheet material.

The number of these mating male and female portions on adjacent zig-zag rings is not limited to the number shown in Fig. 6. The ratio of mating portions to voids, i.e. points at axial ends of the rings at which the bridge struts 14A, 14B are cut-off during the laser-cutting process, can be as much as 1 to 5, or even 1 to 6 depending on the design of the mesh structure used for the stent. It goes without saying that the number of male portions corresponds to the number of female portions. The number, however, can be readily changed during manufacture of the stent tube.

It has been found that heat generated during the laser-cutting process oxidises part of the metal surface of both male and female form-fitting portions, so that both portions are electrically insulated from one another in the assembled state. This oxide layer provides a portion of reduced or virtually zero electrical conductivity that is effective to improve MRI-imaging of the stent lumen.

The technology described in WO 03/075797, and abstracted in the passages above, can be adapted to the provision of inter-loop and intra-loop connection within embodiments of the present invention.

The skilled reader will appreciate that other or additional ways of providing reduced conductivity portions intermediate between the two mating portions of two adjacent rings are conceivable, such as immersing either one or both of the mating portions into an oxidising agent or radiating one or both of the mating portions with a laser, thereby generating sufficient heat to oxidise their metal surfaces. It is conceivable that the naturally occurring oxide layer on the surface of the metal stent might be sufficient for providing the conductivity break, especially when the two mating portions are not in physical contact with each other, such that a small gap exists therebetween.

The thickness of a laser-generated oxide layer depends on the time period and the intensity of the laser used for radiating one of the mating portions. The thickness of this oxide layer should be sufficient that, when the current induced by the external magnetic field exceeds a certain level, a current-breakthrough between two adjacent rings does not occur.

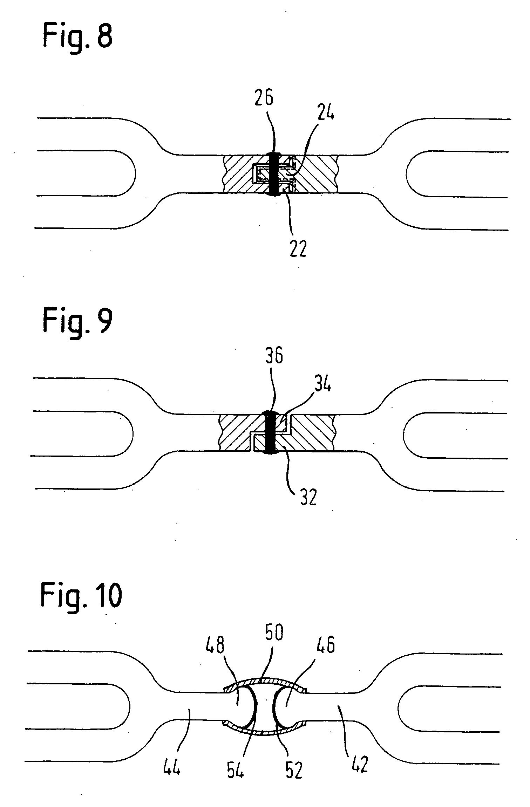

The skilled reader will also appreciate that other ways of connecting two adjacent rings are realisable. Those alternatives include hook and eye and plug-and-socket type connections, spigot-shoulder type connections, bolt-sleeve type connections, clamped arrangements, glue-type connections, hinge-type connections which further enhance axial flexibility of the stent tube, thread-eyelet type connections in which a thread is fed through respective eyelets at axial ends of the rings and subsequently, the two ends of the thread are knotted to the eyelets of the rings for holding the rings together. It is also conceivable using sleeves for connecting axially protruding bridge struts of two adjacent rings, thereby providing a stent structure in which there is no axial connection of two adjacent rings except by the sleeves. The sleeves can be made of a material having low electrical conductivity. The protruding bridge struts of two adjacent rings may comprise the shape of a bone structure, i.e. the diameter of the protruding portion increases towards its axial end. See Fig. 10, described below.

When inserting the male form-fitting portion into the female form-fitting portion, these two portions stay together upon radial expansion of the stent tube solely due to their complementary form-fit. The male portion is inserted into the female portion radially inwardly due to their radially tapered shape, so that upon radial expansion of the stenting rings, the female portion can push the male portion radially outwardly, thereby pressing the male head portion further inwardly into the female recess portion against the rebated internal abutment surface of the female portion. Friction between the complementary male and female portions may help to improve the rigidity of the connection (see WO 02/15820). However, this effect is more amenable to application in balloon-expandable stents, than it is in self-expandable stents. In self-expandable stents, upon deployment of the stent by proximal progressive withdrawal of an outer confining sheath, the angle between the released and unreleased portion of the stent can be large enough to spring the male-female bridge strut engagement apart, at the moment of release from the sheath.

A biocompatible adhesive, although not necessary, may be used to permanently attach two adjacent rings with one another. If the biocompatible adhesive is moreover non-conductive, the extra oxide layer created by, e.g. immersing at least one of the ends of the two complementary form-fitting portions into an oxidising agent, may be omitted. Suitable adhesives may include polymeric based adhesives, such as parylene, acrylate, silicone, PTFE, and stable or biodegradable adhesives. An example of biodegradable adhesives includes lactide acids. Biodegradable adhesives are thought of being advantageous in that they render the stent structure more flexible after deployment and once the process of biodegradation has started. It is also contemplated coating the axially protruding bridge struts with a non-conductive coating. Suitable coatings include diamond-like carbon (DLC) coatings, SiC, SiO₂ or ceramic coatings.

Linkage between two adjacent rings via connecting two bridge struts facing each other can be obtained by using the adhesive or coating itself as the linking member, or by bringing the bridge struts in close proximity with each other so that a gap remains therebetween, e.g. using a sleeve, thereby ensuring that no direct contact between the bridge ends is established, neither within nor outside of the sleeve. However, the latter does not exclude that an adhesive or coating is applied to the thus connected bridge ends.

Methods of applying an adhesive and/or coating include physical vapor deposition (PVD), implantation, injection, dipping, welding, soldering, brazing, plasma deposition, flame-spraying etc.

The skilled person, however, will appreciate that other adhesives and coatings, and methods of applying them, are conceivable.

The junction between two adjacent stenting rings, or even the adhesive or coating itself, may be used as a carrier for drugs inhibiting restenosis. The drugs can be incorporated into the adhesive and/or coating, and will be released therefrom in a dosed manner, so that restenosis is prevented from occurring inside the lumen of the stent.

In Fig. 7, the two stenting rings are illustrated in the disassembled state. As can be seen, the two male and female complementary form-fitting portions are capable of snugly fitting together with reduced conductivity in between. The luminal surface of the bridges 12 is flush with the luminal surface of the stenting rings. This, however, is not crucial for carrying the inventive concept into effect. The luminal surface of the bridges may well be located radially inwardly with respect to the luminal surface of the stenting rings. However, in order to provide unobstructed fluid flow through the stent lumen, the luminal surfaces of the bridges should preferably be flush with the luminal surfaces of the rings.

Fig. 8 shows a connecting bridge between two connected stenting rings with male and female complementary form-fitting portions forming the bridge between two stenting rings according to another preferred embodiment of the invention. The female form-fitting portion has the shape of a fork 22 receiving the male form-fitting portion 24 within the recess in the centre of the fork. Due to the laser cutting process, both male and female form-fitting portions are frusto-conically shaped. There is a gap between the male and female form-fitting portion. If a laser is used for cutting, the size of the male and female form-fitting portions essentially corresponds to the dimension of the laser beam focus. The male and female form-fitting portions can be produced, however, separately, in which case the gap therebetween may differ from the dimension of the laser focus. This gap accounts for enhanced flexibility of this type of structure. A laser-drilled through-hole extends through the male and female form-fitting portions such that both through-holes are in line in order to allow a pin 26 to be inserted therethrough for fixation of the male form-fitting portion to the female form-fitting portion. The through-holes can be created by a laser beam drill, either under manual control under a microscope, or automatically under microprocessor control. Preferably, the pin has a surface made of an electrically-insulating material, such as an oxide layer. It is also contemplated to use pins 26 fabricated entirely from non-conductive material, such as polymeric based materials, ceramics, etc.

Fig. 9 shows another preferred embodiment of the connecting bridge used in the invention of the present application. Two stenting rings are connected via mating portions 32, 34, both stenting rings are complementary in shape and have a through-hole through which a pin 36 can be inserted so that the bridge functions as a hinge joint. Again, due to the laser beam focus having a finite width, a gap remains between the two complementary portions when connected, so that the connection allows a certain degree of pivotal movement when the stent tube is advanced along a tortuous path inside a body vessel.

Each hinge pin 26, 36 may be mechanically fixed to the respective ends of the two complementary mating portions, such as by glueing, or may be fixed in some other way. Again, the cylindrical surface of the pin is preferably electrically-insulating.

Fig. 10 shows bridge struts 42, 44 provided with a bulbous cantilever end, 46, 48 respectively, and surrounded by a shrink sleeve 50. Each of the bulbous ends is treated to provide it with an insulating oxide layer 52, 54. The bridge functions somewhat like a knee joint.

The skilled reader is to understand that the various electrically-insulating joint possibilities described above and in Figs. 6 to 10 and in Applicant's earlier WO 02/047575 are available for application to any of the locations in any of the implants described above, and with reference to Figs. 1 to 5 of the accompanying drawings. Amongst these further possibilities are:
i) mechanical friction fit arrangements, such as an undersize press fit, use of a thermal expansion effect, spring effects, use of coatings and additives to change surface friction properties;
ii) mechanical interlock arrangements, such as use of taper surfaces for nested arrangements or plastic deformation of portions, such as by twisting, to interlock portions together;
iii) use of additional joint component part, such as a micromoulded plastics component to be heat staked or ultrasonically welded, laser welded or friction fitted in place, or use of polymer strands, fibers, fibrils or powder or film to join adjacent metal surfaces;
iv) encapsulate the adjacent metal portions at the joint, as by over-moulding or other use of a containing form around the joint, or by coating the joint with powder and then subjecting it to laser sintering, sintering and surface fusing of a ceramic powder or curing of a resin by a laser in a bath of the resin;
v) high resistance spot welding at the joint;
vi) joining by brazing with a ceramic filler;
vii) doping the adjacent loop portion to be bonded together at the joint.

Within the scope of the present invention we contemplate a tubular implant built up from a sequence of three annuli in which the middle annulus (what one might term the "filling" in the "sandwich") is of electrically insulating material and the annuli radially inside and outside the middle annulus are of electrically conductive material. In this way, the outer annuli contain the conductive paths needed for creation of the balanced coils of the present invention, while the middle annulus provides the insulating mechanical connections between electrically separate conductive paths. We envisage conductive bridges through the insulating annulus to conduct electrically conductive path portions within any one balanced coil.

We envisage various ways of building the sandwich construction of three annuli. One may start from flat stock, create in it a network of conductive bridges, then roll up the device thus prepared, to create a tubular device in accordance with the present invention. Otherwise, one may start from tubular stock and create within it the required balanced coils by known techniques such a laser cutting or chemical etching. See, for example, WO 96/033672 for an example of two conductive annuli separated by an annulus of expanded polytetrafluoroethylene.

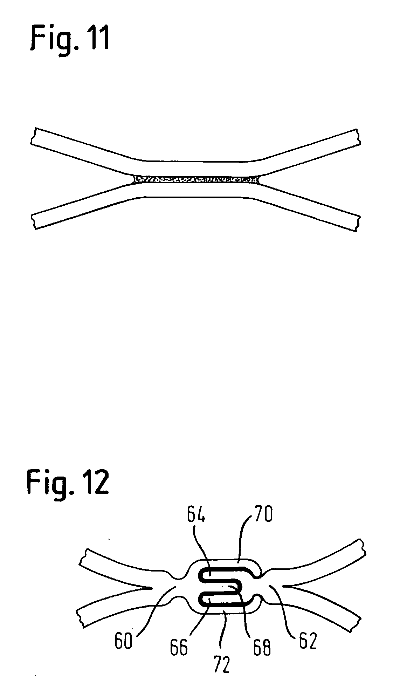

Drawing figures 12 to 24 reveal further ideas for joint constructions, these being proposed specifically to make the joints in constructions such as the embodiment of Fig. 5, in which two "categories" of joint can be perceived. Referring to Fig. 5, and moving along the horizontal axis between 0° to 360°, we find eight rows of joints spaced at 45° angles around the 360° circumference of the stent. Each of these rows features both kinds of joint. Joints 150A lie between side-by-side adjacent portions of either two different closed loops or two portions of the same closed loop within the stent matrix. By contrast, each joint 150B is between nose-to-nose facing vertices of adjacent zig-zag stenting rings. A joint of the category 150B, between nose-to-nose vertices of next adjacent stenting rings, is a regular feature of stents made up of zig-zag stenting rings and such joints are discussed in the context of present Figs. 6 to 10.

Joints of category 150A between side-by-side struts are rather different, at least in regard to the pattern of stress that such a joint will experience during deployment and use of the implant. Accordingly, there is room for fresh thinking how such joints might most attractively be fashioned.

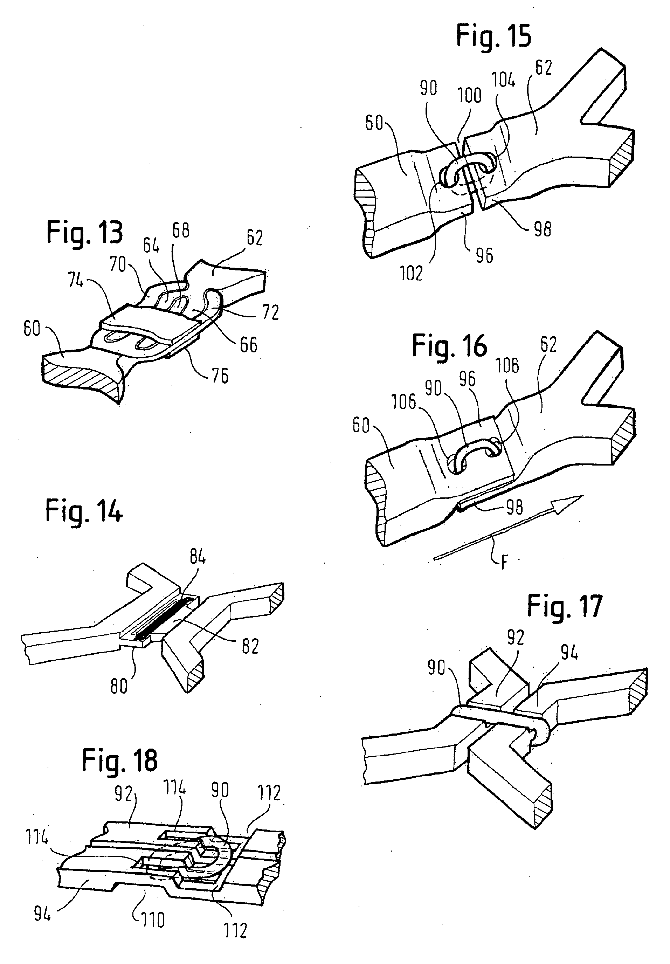

Turning now to the further drawing figures, some of the earlier stated joints are apt for nose-to-nose connections like 150B and others are more appropriate for a side-by-side connection like 150C in Fig. 5. Thus, nose-to-nose connections are shown in drawing Figs. 12 to 16, 19 to 21B and Fig. 24 whereas side-by-side joint connections can be seen in drawing Figs. 14, 17, 18, 22 and 23.

One of the stimuli for the development of new joint structures is the perception that the longevity and properties of adhesives are unpredictable. Any likelihood that a device may become disassembled after deployment in the body is adverse. This is particular difficult if, upon such disassembly, parts of the implant move with respect to the other in such a way that the lumen within which the implant has been deployed might then become occluded by any part of the disassembled device.

However, in order to make available effective joints that do not rely (solely) on adhesive like, for example, the joint illustrated in Fig. 11, some degree of three-dimensionality is indicated. The present applicant is particularly interested in implants made of nickel titanium shape memory alloy. Initial experiments suggest that an implant with a wall-thickness of 240 µm could be locally thinned or compressed, for formulating a joint, down to a thickness of around 100 µm. This opens up numerous possibilities, as we see below.

Turning first to Fig. 12, between the facing noses 60, 62 of adjacent zig-zag stenting rings, a joint is provided that places parallel fingers 64, 66 on nose 62 into the interdigital spaces between three parallel fingers 68, 70, 72 on nose 60. It will be appreciated that such a structure multiplies the surface area of adhesive bonding between the noses 60 and 62, for any given gap length between these two noses.

Furthermore, adhesive joints are relatively weak in peeling mode but the interlocking fingers design of Figs. 12 and 13 is relatively resistant to any peeling because the sort of stresses that would lead to peeling are resisted by the outer fingers 70 and 72 which encapsulate the adhesive bonded surfaces.

However, for greater security against disassembly, a retaining strap 74 on the abluminal surface of the joint and a similar strap 76 on the luminal surface of the joint can be added. The material of the zig-zag stenting rings can be made thinner between the noses 60 and 62, in the vicinity of the interlocking fingers, in order to accommodate straps 74 and 76 within more or less the general wall thickness of the device. As to electrical insulation, it will be appreciated that the interlocking surfaces of the fingers can be electrically isolated from the fingers of the other component. As to the straps 74, 76, these could be of metal and welded to the outer fingers 70 and 72, and could even be welded to the middle finger 68 while at the same time denying electrical contact between either strap 74, 76 and either finger 64, 66. One envisages, for surface smoothness, an overcoating of formable material, between the noses 60 and 62 and around the straps 74 and 76, to achieve a smooth surface contour, and to enhance the strength of the joint.

Turning to Fig. 14, an analogous joint for a side-to-side junction, rather than nose-to-nose junction, is illustrated. Material protrudes from the facing surfaces of parallel adjacent portions of the implant, and features a wall thickness somewhat less than the general wall thickness of the implant. The protruding portions feature an interlocking dovetail joint between portions 80 and 82 (and it will be appreciated that the surfaces of the dovetail are treated so as to deny electrical conductivity across the dovetail joint). As in Fig. 13, a strap 84 can be provided across the dovetail joint on the abluminal surface of the device and a similar strap (not shown) can be provided on the luminal surface, thereby denying any possibility for the male part 82 of the dovetail to slide out of the female portion of the dovetail, in a direction upwardly or downwardly as seen in Fig. 14. Again, like in Fig. 13, the joint could be "potted" in a formable material which is electrically-insulating, in order to confirm and strengthen the integrity of the joint.

Moving on to drawing Figs. 15 to 18, these show variations on a theme of using a cord 90 to connect nose-to-nose as shown in Figs. 15 and 16, or side to side portions as shown in Figs. 17 and 18. In Fig. 15, locally thinned or compressed portions 96, 98 are arranged in close end-to-end proximity, with the cord 90 in the form of a closed loop bridging the gap 100 between the nose-to-nose implant parts. The cord is conveniently of a high-strength polymer can be welded so as to form the closed loop after the cord has been threaded through bores 102 and 104 in thinned portions 96 and 98 respectively. As with the embodiments described above, the entire joint area between the noses 60 and 62 can be "potted" with an electrically insulating polymer material to strengthen the joint and confirm the electrical isolation between noses 60 and 62.

In Fig. 16, each thinned portion 96, 98 is provided with a pair of bores 106, 108 with like spacing so they can be arranged to line up in order for the cord 90 to be passed through both bores 106 and then both bores 108 before being joined end-to-end to form the closed loop of cord which holds the two noses 60 and 62 joined together. Again, the joined area can be potted in formable polymer material to maintain electrical isolation and integrity of the joint.

Of note is direction F as indicated by the arrow in Fig. 16. Looking at Fig. 16, the abluminal surface of the implant is the upper surface seen in the drawing. Arrow F indicates the direction of deployment of the implant (by withdrawal of a confining sheath in a direction opposite arrow F), with the consequence that the zigzag stenting ring which includes nose 62 is released by the retreating sheath before the sheath releases zigzag ring which includes nose 60. In consequence, the tendency of nose 62 to expand radially away from nose 60 is resisted by thin portion 96 overlying thin portion 98, radially outside it. Thus, the stresses carried by the joint during deployment of the implant are carried by the metal of the zigzag rings rather than by the polymer of the cord 90. It will be appreciated that, if thin portion 98 were to be overlying thin portion 96 in Fig. 16, and then the sheath were to be withdrawn from right to left in Fig. 16, with the implant being deployed in the direction shown by arrow F, then it would fall to the cord 90 to retain the integrity of the joint as nose 62 seeks to move upwardly (radially outwardly) relative to thin portion 96.

Provided that the implant is built is such a way as to play to the inherent strength of the lap joint shown Fig. 16, this should be more resilient to the forces carried by the implant during its deployment and loading than the butt joint of Fig. 15. In both cases, it is envisaged that the local thinning of the implant wall thickness for portions 96 and 98 would be from 250 µm by around 50 µm on each side of the centreline of the wall thickness, with then a cord of 50 µm diameter serving to join the two thinned portions. However, naturally, dimensions would be selected which are apt for the particular implant being designed. As to the lap joint of Fig. 16, one envisages thinned portions lapping over each other that are each of a thickness of about 50 µm, for a general implant wall thickness of 250 µm.

Looking now at cord systems for joining side-by-side portions of the implant, we see in Fig. 17 a cord 90 joined as by welding to formed a closed loop which sits in a locally formed recess into adjacent side-by-side portions 92 and 94 of the implant. Typically, the implant has a wall thickness of 250 µm, the cord a diameter of 50 µm, and the recesses to receive the cord being of appropriate dimensions to accommodate the cord snugly. Again, the joint area would be filled with adhesive or other polymer formable composition, to confirm electrical isolation between portions 92 and 94 and to improve the strength of the joint. It will be appreciated that the cord need not be of electrically insulating material, provided insulation can be achieved between the conductive cord 90 and each of the side-by-side portions 92 and 94.

Fig. 18 offers a possibility to use cord 90 of greater diameter and therefore greater strength. Each of the side-by-side portions 92 and 94 is formed with a recess 110 in its luminal surface and a staggered recess 112 in its abluminal surface, there being an elongate slot 114 through the wall thickness of the implant and extending into each of the recesses 110, 112. In this way, as seen in Fig. 18, there is room within the overall wall thickness for a cord 90 even of a diameter of around 100 µm without leaving unacceptably weakened the mechanical strength of the side-by-side portions 92 and 94 in the joint area. Again, once the joint has been fashioned, the joint area can be filled with electrically insulating formable material to maintain electrical isolation between portions 92 and 94, and enhance the strength and integrity of the joint.

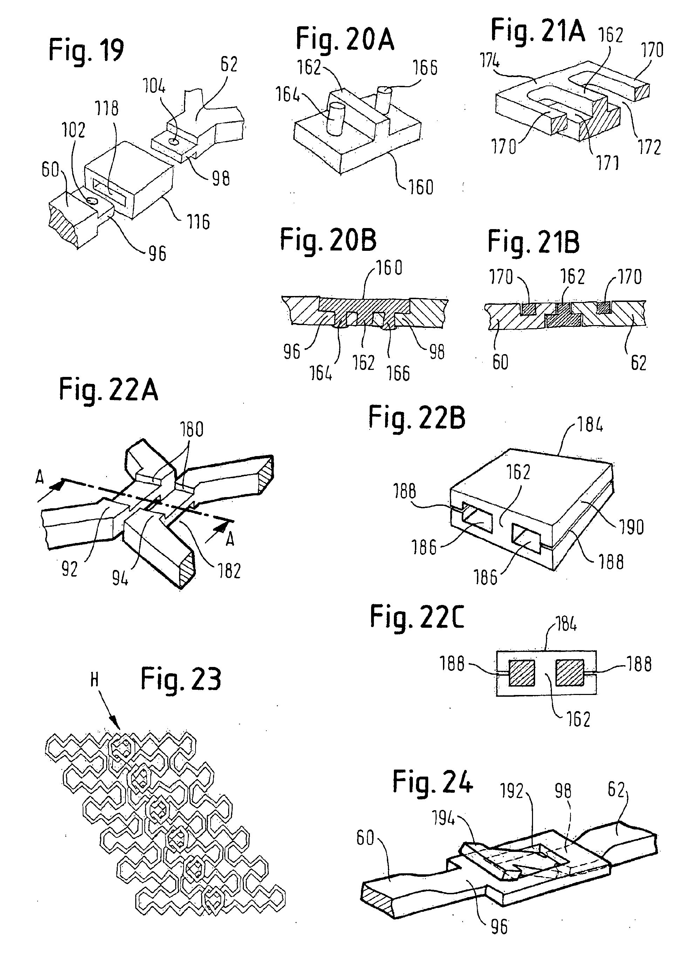

Fig. 19 offers an elegantly simple alternative to the cord 90 of Fig. 15. Instead, a formed joining piece 116 with a through-going slot 118 (or two blind slots separated by a web) to receive the thin portions 96 and 98 can be offered up to the facing noses 60 and 62, and then heated such that material of the joining piece 116 flows into the respective holes 102 and 104 in the thin portions 96 and 98. Upon cooling the joining piece 116, a mechanical interlock is achieved, which has high electrical isolation between the noses 60 and 62.

Figs. 20A and 20B show an alternative connecting piece 160 which features a central abutment 162 and a pair of prongs 164, 166, one each side of the central abutment. Each prong receives a respective one of the holes 102, 104 in relatively thinner portions 96, 98 and, when the thin portions are seated firmly on the connector piece 160, the respective heads of the prongs 164, 166 can be pressed and heated so that the heads overlie the adjacent flat surface of the thinned portions 96 and 98, thereby to function in the nature of rivets.

The embodiment of Figs. 21A and 21B is effective without heat-forming. Rather, each nose 60 and 62 is provided with respective recesses in its luminal and abluminal surface to receive corresponding beam portions 171, 172, each side of a central divider 162 in a connecting piece 174 which evidently functions much in the nature of an office paper clip to retain in a desired end-to-end configuration the two noses 60 and 62.

As to a joint between two side-by-side extending portions, analogous to Fig. 17, consider Figs. 22A, B and C. The two portions 92 and 94 are locally thinned on the abluminal 180 and luminal 182 major surfaces of the implant, to accommodate the wall thickness of a connector piece 184 around a pair of parallel bores 186 which run the length of the connector piece 184. Between each bore 186 and the corresponding side wall surface 190 of the connector piece 184 is a slit 188 so that deformation of the material of the connector piece 184, each side of the central isolating web 162, allows the connector piece to be eased over the corresponding thinned portions 180, 182 of the respective side-by-side portions 92 and 94 of the implant. It will be appreciated that the connector piece 184 can be regarded as a "double-C-piece". The open ends of each C, that is to say slits 188, can be closed by conventional methods such as heat staking, laser welding or ultrasonic welding. Alternatively, an over-molding technique could be employed to form a connector piece such as is indicated in Figs. 22B and C, without ever needing to provide any slits 188.

Finally, mechanical interlocks are suggested in drawing Figs. 23 and 24. Drawing Fig. 23 shows interlocking of two adjacent "winding loops" of this invention. Avoidance of adhesive and polymer materials is achieved at the expense of a helical zone H of overlap between two adjacent winding loops which are interlocked and lie over each other in a double thickness around the helix H. Fig. 24 avoids any such double thickness, by providing on one thin nose 96 a rectangular surface 192 with its longer length direction extending towards the other part of the joint on the facing nose 98. The thinned portion 98 of the second nose piece 62 features a T-shape or hammer head joint portion 192 that cooperates with the orifice 192. The length of the crosspiece of the T-shape is greater than the width of the orifice 192, but not as great as the length of the orifice 192. Accordingly, the T-piece can be passed through the orifice 192, and then rotated into the locking configuration shown in Fig. 24. Again, the integrity of joint is not dependent upon polymers or adhesives although, again, maintenance of electrical isolation between portions 96 and 98 dictates that an electrically insulating barrier be placed between these two portions. As with other illustrated embodiments, the joint area can be filled or potted with formable polymeric electrically insulating material, if not for joint strengthening and enhanced electric isolation, then for enhanced surface smoothness which is generally desirable for any implant which is to be deployed within the body.

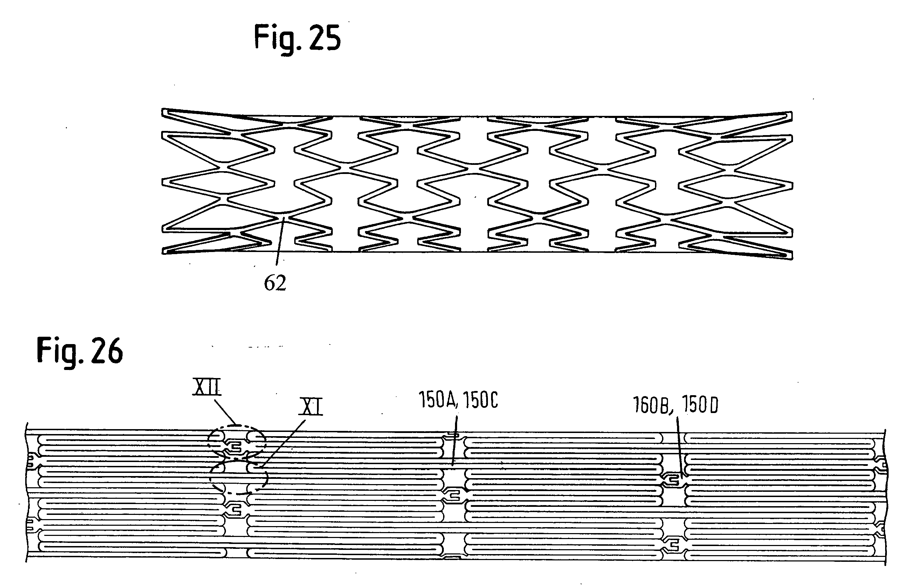

The inventors have built and tested an embodiment of the present invention that corresponds to the matrix shown in Fig. 5. The test results vindicated the promise of the invention. The lumen of the stent matrix cylinder was visible in MRI images. The construction of the tested embodiment is revealed in Figs. 25 and 26, and is described below.

Applicant's WO 01/32102 discloses laser cut stents of nickel titanium shape memory alloy with stenting rings spaced apart along the length of the stent, and joined together by conductive bridges, as seen as feature 62 in Fig. 25. In Fig. 26, a similar structure is evident, except that some of these bridges are severed lengthwise, and some transversely, to create the winding coils evident in Fig. 5 described above. The severance is accomplished by the same laser that creates the stent strut matrix from a plain NITINOL tube stent precursor workpiece.

The bridges cut lengthwise are as in Fig. 17, except that the function of the band 90 is performed, in the testpiece, by epoxy adhesive. The bridges cut transversely resemble the joint described above with reference to Fig. 12. Again, epoxy adhesive was used in the testpiece to bond together, yet electrically insulate, the two co-operating portions that form the joint of Fig. 12. The tested stent matrix exhibited 2 winding coils, each extending the full length of the stent matrix, and each exhibiting an even number of lobes that, in aggregate, define equal and opposite areas cut by an incident B₁ field so that the aggregate flow of eddy currents in each such winding coil is essentially zero.

The embodiments described above with reference to the drawings are to be understood as examples of constructions within the scope of the claims which follow, and of the inventive concepts disclosed above.

## Claims

1. An implant (100) comprising:
electrically-conductive closed loops (300A, 300B, 300C, 300D) forming an apertured wall of the implant with an interior volume, each of said loops being formed from loop portions providing electrically- conductive current pathways within which eddy currents are liable to be induced when subjected to a time-dependent external magnetic field, each of said loops consists of a first current pathway and a second current pathway wherein said first current pathway and said second current pathway are arranged such that, regardless of the direction of said external magnetic field, the direction of the eddy current that would be induced by said field in said second current pathway is the reverse of the direction of the eddy current that would simultaneously be induced by said field in said first current pathway, thereby to prevent flow of eddy currents in each of said loops.

2. The implant according to claim 1, wherein each of said loops has loop portions formed as a first lobe (I) and as a second lobe (II) of a figure of eight, and further compromises a cross-over point between said first lobe and said second lobe.

3. The implant according to claim 2, further compromising an electrically-insulating joint between said two loop portions at said cross-over point.

4. The implant according to claim 2 or 3, wherein each of said loops has additional lobes and additional cross-over points (600) between said additional lobes, with the areas bounded by the lobes being such that, in aggregate, the area bounded by one set of lobes equals the area bounded by a cancelling remainder of the lobes.

5. The implant according to any one of the preceding claims, wherein the implant has a central longitudinal axis and said interior volume is tubular and centred on said axis.

6. The implant according to claim 5, wherein each of said loops wraps around said axis in the form of a spiral with an integral whole number of turns.

7. The Implant as claimed in claim 6, the integral whole number of turns being at least three.

8. The implant according to claim 6 or 7, wherein each of said loops that wraps around the axis lies within an envelope that is transverse to the axis.

9. The implant according to claim 6,7 or 8, wherein each of said loops wraps around the axis in a path that spirals around the axis from one end of the implant to the other.

10. The implant according to any one of claims 6 to 9, wherein the pitch of said spiral path is constant.

11. The implant according to any one of the preceding claims, wherein loop portions correspond to struts that are joined end-to-end to each other and can deploy in use to form a zig-zag pattern.

12. The implant according to any one of the preceding claims, with the plurality of loops arranged mutually axially adjacent, and spaced along the axis.

13. The implant according to claim 12, wherein adjacent loops are connected to each other by electrically-insulating links (800).

14. The implant according to any one of the preceding claims, wherein each of said loops includes a plurality of electrically-insulating links (600) that connect spaced loop portions of said loop.

15. The implant according to claim 13 or 14, wherein each link is a mechanical coupling with a first cooperating link portion and a second cooperating link portion.

16. The implant according to claim 15, wherein the cooperating portions can move relative to each other.

17. The implant according to claim 16, wherein the cooperating portions are constituted as a hook portion and an eye to receive the hook portion.

18. The implant according to any one of claims 15, 16 and 17, and including a layer of bonding material between the cooperating link portions.

19. The implant according to claim 18, wherein the bonding material is ceramic.

20. The implant according to claim 18, wherein the bonding material is an adhesive composition.

21. The implant according to any one of claims 15 to 20, wherein the mechanical coupling comprises interlocking fingers.

22. The implant according to any one of claims 15 to 21, wherein the mechanical coupling comprises mechanically-engaging surfaces in combination with at least one restraining strap overlying the engaging surfaces.

23. The implant according to any one of claims 13 to 22, wherein each link includes a molded connector piece.

24. The implant according to any one of claims 13 to 23, wherein each link includes a portion that is locally thinned with respect to the thickness of the wall of the implant.

25. The implant according to any one of the preceding claims, in which the wall of the implant is an apertured tube.

26. The implant according to any one of the preceding claims, wherein the implant is made of nickel-titanium shape memory alloy.

27. The implant according to any one of claims 1 to 25, wherein the implant is made of stainless steel.

28. The implant according to any one of the preceding claims, wherein the implant is a stent.

29. The implant according to claim 28, wherein the stent is radially expansible from a radially compact delivery configuration to a radially larger deployed configuration, and the stent is capable of being delivered transluminally by a catheter.

30. The implant according to any one of claims 1 to 27, wherein the implant is a filter.

31. The implant according to any one of claims 1 to 27, wherein the implant is a valve.

32. The implant according to any one of claims 1 to 29, wherein the implant is a graft.

33. The implant according to any one of the preceding claims, wherein the implant is a self-expanding implant delivered transluminally in a radially compact configuration and capable of self-expansion into a radially larger deployed configuration at an implant site.

34. The implant according to any one of claims 1 to 3, wherein each closed loop exhibits lobes, with an equal lobe area on opposite sides of the interior volume.

35. An implant tube comprising: an electrical conductor, said electrical conductor having a plurality of closed loops electrically insulated from each other, each of said closed loops having a periphery of a string of equal area lobes that are within said closed loop, and every one of said lobes has a counterpart lobe of equal area located diametrically opposite on the implant tube.

36. The implant tube according to claim 35, wherein each of said loop having an even number of lobes.

## Patentansprüche

1. Implantat (100), aufweisend:
elektrisch leitende, geschlossene Schlingen (300A, 300B, 300C, 300D), die eine geöffnete Wand des Implantats mit einem inneren Volumen ausbilden, wobei jede der Schlingen aus Schlingenabschnitten ausgebildet ist, die elektrisch leitende Strompfade bereitstellen, innerhalb derer Wirbelströme entsprechend induziert werden, wenn einem zeitabhängigen externen Magnetfeld ausgesetzt, wobei jede der Schlingen einen ersten Strompfad und einen zweiten Strompfad aufweist, wobei der erste Strompfad und der zweite Strompfad derart angeordnet sind, dass, unabhängig von der Richtung des externen Magnetfelds, die Richtung des Wirbelstroms, der durch das Feld im zweiten Strompfad induziert würde, umgekehrt der Richtung des Wirbelstroms ist, der gleichzeitig durch das Feld im ersten Strompfad induziert würde, wodurch ein Strom der Wirbelströme in jeder der Schlingen verhindert wird.

2. Implantat gemäß Anspruch 1, bei dem jede der Schlingen Schlingenabschnitte aufweist, die als ein erster Lappen (I) und als ein zweiter Lappen (II) einer Darstellung einer Acht ausgebildet sind, und ferner einen Kreuzungspunkt zwischen dem ersten Lappen und dem zweiten Lappen aufweist.

3. Implantat gemäß Anspruch 2, ferner umfassend eine elektrisch isolierende Verbindung zwischen zwei Schlingenabschnitten am Kreuzungspunkt.

4. Implantat gemäß Anspruch 2 oder 3, bei dem jede der Schlingen zusätzliche Lappen und zusätzliche Kreuzungspunkte (600) zwischen den zusätzlichen Lappen aufweist, wobei die Gebiete, die durch die Lappen abgegrenzt sind, derart sind, dass, im Ganzen, das durch einen Satz von Lappen abgegrenzte Gebiet dem Gebiet gleicht, das durch Streichen von verbleibenden der Lappen abgegrenzt ist.

5. Implantat gemäß einem der vorangegangenen Ansprüche, bei dem das Implantat eine Mittenlängsachse aufweist und das innere Volumen röhrenförmig und an der Achse zentriert ist.

6. Implantat gemäß Anspruch 5, bei dem sich jede der Schlingen um die Achse in der Gestalt einer Spirale mit einer integralen ganzen Zahl von Windungen wickelt.

7. Implantat gemäß Anspruch 6, wobei die integrale ganze Zahl von Windungen zumindest drei ist.

8. Implantat gemäß Anspruch 6 oder 7, bei dem jede der Schlingen, die sich um die Achse wickelt, innerhalb einer Hülle liegt, die quer zur Achse befindlich ist.

9. Implantat gemäß Anspruch 6, 7 oder 8, bei dem sich jede der Schlingen in einem Pfad um die Achse wickelt, die sich vom einen Ende des Implantats zum anderen spiralförmig um die Achse windet.

10. Implantat gemäß einem der Ansprüche 6 bis 9, bei dem die Steigung des Spiralenpfads konstant ist.

11. Implantat gemäß einem der vorangegangenen Ansprüche, bei dem Schlingenabschnitte Streben entsprechen, die an Enden miteinander verbunden sind und sich im Gebrauch entfalten können, um ein Zick-Zack-Muster auszubilden.

12. Implantat gemäß einem der vorangegangenen Ansprüche, mit einer Vielzahl von Schlingen, die wechselweise axial benachbart angeordnet sind, und entlang der Achse beabstandet sind.

13. Implantat gemäß Anspruch 12, bei dem benachbarte Schlingen durch elektrisch isolierende Verknüpfungen (800) miteinander verbunden sind.

14. Implantat gemäß einem der vorangegangenen Ansprüche, bei dem jede der Schlingen eine Vielzahl von elektrisch isolierenden Verknüpfungen (600) aufweist, die beabstandete Schlingenabschnitte der Schlinge verbinden.

15. Implantat gemäß Anspruch 13 oder 14, bei dem jede Verknüpfung eine mechanische Kopplung mit einem ersten kooperierenden Verknüpfungsabschnitt und einem zweiten kooperierenden Verknüpfungsabschnitt ist.

16. Implantat gemäß Anspruch 15, bei dem sich die kooperierenden Abschnitte relativ zueinander bewegen können.

17. Implantat gemäß Anspruch 16, bei dem die kooperierenden Abschnitte als ein Hakenabschnitt und ein Auge zum Empfangen des Hakenabschnitts gebildet sind.

18. Implantat gemäß einem der Ansprüche 15, 16 und 17, und aufweisend eine Lage eines Anbindungsmaterials zwischen den kooperierenden Verknüpfungsabschnitten.

19. Implantat gemäß Anspruch 18, bei dem das Anbindungsmaterial eine Keramik ist.

20. Implantat gemäß Anspruch 18, bei dem das Anbindungsmaterial eine Haftmittelzusammensetzung ist.

21. Implantat gemäß einem der Ansprüche 15 bis 20, bei dem die mechanische Kopplung verriegelnde Finger umfasst.

22. Implantat gemäß einem der Ansprüche 15 bis 21, bei dem die mechanische Kopplung mechanische Eingriffsflächen in Kombination mit zumindest einem zurückhaltenden Band umfasst, das über den Eingriffsflächen liegt.

23. Implantat gemäß einem der Ansprüche 13 bis 22, bei dem jede Verknüpfung ein gegossenes Verbinderstück umfasst.

24. Implantat gemäß einem der Ansprüche 13 bis 23, bei dem jede Verknüpfung einen Abschnitt umfasst, der bezüglich der Dicke der Wand des Implantats lokal dünner gemacht ist.

25. Implantat gemäß einem der vorangegangenen Ansprüche, bei dem die Wand des Implantats ein offenes Rohr ist.

26. Implantat gemäß einem der vorangegangenen Ansprüche, bei dem das Implantat aus einer Nickel-Titan-Formgedächtnislegierung gefertigt ist.

27. Implantat gemäß einem der Ansprüche 1 bis 25, bei dem das Implantat aus Edelstahl gefertigt ist.

28. Implantat gemäß einem der vorangegangenen Ansprüche, bei dem das Implantat ein Stent ist.

29. Implantat gemäß Anspruch 28, bei dem der Stent von einem radial kompakten Zuführaufbau zu einem radial größeren entfalteten Aufbau radial expandierbar ist, und der Stent transluminal durch einen Katheter zugeführt werden kann.

30. Implantat gemäß einem der Ansprüche 1 bis 27, bei dem das Implantat ein Filter ist.

31. Implantat gemäß einem der Ansprüche 1 bis 27, bei dem das Implantat ein Ventil ist.

32. Implantat gemäß einem der Ansprüche 1 bis 29, bei dem das Implantat ein Pfropfen ist.

33. Implantat gemäß einem der vorangegangenen Ansprüche, bei dem das Implantat ein selbst-expandierendes Implantat ist, das in einem radial kompakten Aufbau transluminal zugeführt wird und am Implantationsort eine Selbstexpansion in einen radial größeren, entfalteten Aufbau durchführen kann.

34. Implantat gemäß einem der Ansprüche 1 bis 3, bei dem jede der geschlossenen Schlingen Lappen aufweist, mit einem gleichen Lappengebiet an gegenüberliegenden Seiten des inneren Volumens.

35. Implantat-Rohr, aufweisend:
einen elektrischen Leiter, wobei der elektrische Leiter eine Vielzahl von geschlossen Schlingen aufweist, die voneinander elektrisch isoliert sind, wobei jede der geschlossenen Schlingen einen Umfang eines Fadens von Lappen mit gleichem Gebiet aufweist, die sich innerhalb der geschlossenen Schlinge befinden, und jeder der Lappen einen Gegenstücklappen mit gleichem Gebiet aufweist, der am Implantat-Rohr diametral gegenüber gelegen ist.

36. Implantatrohr gemäß Anspruch 35, bei dem jede der Schlingen eine gerade Anzahl von Lappen aufweist.

## Revendications

1. Implant (100) comprenant :
des boucles fermées électriquement conductrices (300A, 300B, 300C, 300D) formant une paroi trouée de l'implant avec un volume intérieur, chacune desdites boucles étant formée de portions de boucle constituant des voies de courant électriquement conductrices à l'intérieur desquelles des courants de Foucault sont susceptibles d'être induits lorsqu'elles sont soumises à un champ magnétique externe dépendant du temps, chacune desdites boucles consistant en une première voie de courant et une deuxième voie de courant, dans lequel ladite première voie de courant et ladite deuxième voie de courant sont agencées de telle sorte que, quelle que soit la direction dudit champ magnétique externe, la direction du courant de Foucault qui serait induit par ledit champ dans ladite deuxième voie de courant est l'inverse de la direction du courant de Foucault qui serait simultanément induit par ledit champ dans ladite première voie de courant, pour empêcher ainsi l'écoulement de courants de Foucault dans chacune desdites boucles.

2. Implant selon la revendication 1, dans lequel chacune desdites boucles a des portions de boucle formées en tant que premier lobe (I) et en tant que deuxième lobe (II) au nombre de huit, et comprenant en outre un point de croisement entre ledit premier lobe et ledit deuxième lobe.

3. Implant selon la revendication 2, comprenant en outre une articulation électriquement isolante entre lesdites deux portions de boucle sur ledit point de croisement.

4. Implant selon la revendication 2 ou 3, dans lequel chacune desdites boucles a des lobes supplémentaires et des points d'articulation supplémentaires (600) entre lesdits lobes supplémentaires, les zones liées par les lobes étant telles que dans l'ensemble, la zone liée par un groupe de lobes égale la zone liée par une suppression du reste des lobes.

5. Implant selon l'une quelconque des revendications précédentes, dans lequel l'implant a un axe central longitudinal et ledit volume intérieur est tubulaire et centré sur ledit axe.

6. Implant selon la revendication 5, dans lequel chacune desdites boucles s'enroule autour dudit axe sous la forme d'une spirale en un nombre entier de tours.

7. Implant selon la revendication 6, le nombre entier de tours étant d'au moins trois.

8. Implant selon la revendication 6 ou 7, dans lequel chacune desdites boucles qui s'enroule autour de l'axe repose dans une enveloppe qui est transversale à l'axe.

9. Implant selon la revendication 6, 7 ou 8, dans lequel chacune desdites boucles s'enroule autour de l'axe dans une voie qui forme une spirale autour de l'axe d'une extrémité de l'implant à l'autre.

10. Implant selon l'une quelconque des revendications 6 à 9, dans lequel le pas de ladite voie de spirale est constant.

11. Implant selon l'une quelconque des revendications précédentes, dans lequel des portions de boucles correspondent à des entretoises qui sont jointes de bout en bout les unes aux autres et peuvent se déployer lors de l'utilisation pour former un schéma en zigzag.

12. Implant selon l'une quelconque des revendications précédentes, dans lequel la pluralité de boucles est agencée réciproquement de façon adjacente sur le plan axial, et espacée le long de l'axe.

13. Implant selon la revendication 12, dans lequel des boucles adjacentes sont reliées les unes aux autres par des liens électriquement isolants (800).

14. Implant selon l'une quelconque des revendications précédentes, dans lequel chacune desdites boucles inclut une pluralité de liens électriquement isolants (600) qui relient des portions de boucle espacées de ladite boucle.

15. Implant selon la revendication 13 ou 14, dans lequel chaque lien est un couplage mécanique avec une première portion de lien de coopération et une deuxième portion de lien de coopération.

16. Implant selon la revendication 15, dans lequel les portions de coopération peuvent se déplacer l'une par rapport à l'autre.

17. Implant selon la revendication 16, dans lequel les portions de coopération sont constituées sous la forme d'une portion en crochet et d'un oeillet pour recevoir la portion en crochet.

18. Implant selon l'une quelconque des revendications 15, 16 et 17, et incluant une couche de matériau de liaison entre les portions de lien de coopération.

19. Implant selon la revendication 18, dans lequel le matériau de liaison est une céramique.

20. Implant selon la revendication 18, dans lequel le matériau de liaison est une composition adhésive.

21. Implant selon l'une quelconque des revendications 15 à 20, dans lequel le couplage mécanique comprend des doigts de verrouillage.

22. Implant selon l'une quelconque des revendications 15 à 21, dans lequel le couplage mécanique comprend des surfaces de mise en prise mécanique en combinaison avec au moins une bande de retenue recouvrant les surfaces de mise en prise.

23. Implant selon l'une quelconque des revendications 13 à 22, dans lequel chaque lien inclut un élément de connecteur moulé.

24. Implant selon l'une quelconque des revendications 13 à 23, dans lequel chaque lien inclut une portion qui est affinée localement par rapport à l'épaisseur de la paroi de l'implant.

25. Implant selon l'une quelconque des revendications précédentes, dans lequel la paroi de l'implant est un tube troué.

26. Implant selon l'une quelconque des revendications précédentes, dans lequel l'implant est constitué d'un alliage à mémoire de forme de nickel-titane.

27. Implant selon l'une quelconque des revendications 1 à 25, dans lequel l'implant est constitué d'acier inoxydable.

28. Implant selon l'une quelconque des revendications précédentes, dans lequel l'implant est un stent.

29. Implant selon la revendication 28, dans lequel le stent peut être expansé radialement à partir d'une configuration de distribution radialement compacte en une configuration déployée radialement plus grande, et le stent peut être distribué par voie transluminale par un cathéter.

30. Implant selon l'une quelconque des revendications 1 à 27, dans lequel l'implant est un filtre.

31. Implant selon l'une quelconque des revendications 1 à 27, dans lequel l'implant est une valve.

32. Implant selon l'une quelconque des revendications 1 à 29, dans lequel l'implant est un greffon.

33. Implant selon l'une quelconque des revendications précédentes, dans lequel l'implant est un implant auto-expansible distribué par voie transluminale dans une configuration radialement compacte, et capable de s'auto-expanser en une configuration radialement plus grande sur un site d'implant.

34. Implant selon l'une quelconque des revendications 1 à 3, dans lequel chaque boucle fermée présente des lobes, une surface de lobe égale se trouvant sur des côtés opposés du volume intérieur.

35. Tube d'implant comprenant :
un conducteur électrique, ledit conducteur électrique ayant une pluralité de boucles fermées isolées électriquement les unes des autres, chacune desdites boucles fermées ayant un pourtour d'une chaîne de lobes de surface égale qui sont dans ladite boucle fermée, et chacun desdits lobes ayant un lobe correspondant de surface égale situé sur un plan diamétralement opposé sur le tube d'implant.

36. Tube d'implant selon la revendication 35, dans lequel chacune desdites boucles a un nombre pair de lobes.
